# EUROPEAN PATENT APPLICATION

(11) **EP 3 404 091 A1**
(43) Date of publication of application: **21.11.2018**
(21) Application number: 17738412.0
(22) Date of filing: 11.01.2017
(51) Int. Cl.: C12M 1/00, C12M 3/00

(54) **CELL CULTURE VESSEL, AND CULTURE MEDIUM EXCHANGE SYSTEM**

(30) Priority: 14.01.2016 JP 2016005056; 21.09.2016 JP 2016184413
(71) Applicant: Olympus Corporation, Hachioji-shi, Tokyo 192-8507 (JP)
(72) Inventor: KIMURA, Hiroyuki, Hachioji-shi Tokyo 192-8507 (JP); MINAMI, Tatsuya, Hachioji-shi Tokyo 192-8507 (JP); MAKARA, Yasunori, Hachioji-shi Tokyo 192-8507 (JP)
(74) Representative: Winter, Brandl, Fürniss, Hübner, Röss, Kaiser, Polte - Partnerschaft mbB
(86) International application number: PCT/JP2017/000569
(87) International publication number: WO 2017/122658

(57) **Abstract**

A cell culture container (100) of the present invention includes, in sequence from above in the gravity direction: a culture-medium holding container (1); a cell holding container (2) that holds cells and a culture medium (A) supplied from the culture-medium holding container (1) through a supply port (4) provided in the top surface thereof; a waste-liquid holding container (3) that holds the culture medium (A) discharged from the cell holding container (2); and a discharge mechanism (5) for discharging the culture medium (A) that has exceeded a predetermined level from the cell holding container (2) to the waste-liquid holding container (3).

## Description

### {Technical Field}

The present invention relates to a cell culture container in which cells can be cultured for an extended period of time and to a culture-medium changing system.

### {Background Art}

In recent years, the demand for cell culturing while maintaining their three-dimensional structures is growing with the development of stem cell research and regenerative medicine. Typically, cells are cultured in an incubator (warm chamber) that can maintain an environment suitable for their growth. A culture container accommodating cells and a culture medium need to be periodically taken out of the incubator for changing the culture medium (for example, see Patent Literature 1).

### {Citation List}

### {Patent Literature}

{PTL 1} Japanese Unexamined Patent Application Publication No. 2010-273603

### {Summary of Invention}

### {Technical Problem}

A culture-medium changing operation imparts a shock to cells, potentially affecting the cells in ways such as destroying the three-dimensional structures of the cells. Hence, it is desirable to culture the cells for an extended period of time in a state in which they are statically placed in the incubator without taking the culture container accommodating the cells and the culture medium out of the incubator to change the culture medium. However, to do so would inevitably lead to degradation of the culture medium, and the progress of the degradation would increase the effect on the cells.

The present invention has been made in view of the above-described circumstances, and an object thereof is to provide a cell culture container and a culture-medium changing system that enable cell culturing for an extended period of time in a state in which they are statically placed in an incubator by decreasing the rate of degradation of the culture medium.

### {Solution to Problem}

To achieve the above-described object, the present invention provides the following solutions.

An aspect of the present invention is a cell culture container including: a culture-medium holding container that holds a culture medium used to culture cells; a cell holding container that has, in the top surface thereof, a supply port through which the culture medium is supplied from the culture-medium holding container and that holds the cells and the culture medium supplied from the culture-medium holding container through the supply port; a waste-liquid holding container that holds the culture medium discharged from the cell holding container; and a discharge mechanism for discharging the culture medium from the cell holding container to the waste-liquid holding container when the culture medium in the cell holding container has exceeded a predetermined level.

According to this aspect, by gradually replacing the degraded culture medium with an unused culture medium, it is possible to decrease the rate of degradation of the culture medium, enabling culturing of the cells for an extended period of time in a state in which the cell culture container is statically placed in the incubator.

In the above-described aspect, the bottom surface of the culture-medium holding container and the top surface of the cell holding container may adjoin each other, forming a boundary surface, and the supply port may open in the boundary surface.

In the above-described aspect, the culture-medium holding container may communicate with the supply port via a tubular member, and the culture-medium holding container may be disposed above the cell holding container in the gravity direction. Because this configuration makes attachment and detachment of the culture-medium holding container easy, it is easy to supply an unused culture medium to the culture-medium holding container. This configuration also enables replacement with another culture-medium holding container that holds an unused culture medium.

In the above-described aspect, the culture-medium holding container may be a bag attachable to and detachable from the supply port.

In the above-described aspect, the cell holding container may have, in the bottom surface thereof, a discharge port through which the culture medium is discharged from the cell holding container to the waste-liquid holding container, and the discharge mechanism may include a flow path projecting from the discharge port to the upper part of the internal space of the cell holding container and opening at a predetermined height. Because this configuration allows only the excess culture medium that has exceeded the predetermined height to be discharged, constantly leaving a certain amount of culture medium, it is possible to prevent a decrease in the amount of the culture medium in the cell holding container or exhaustion of the culture medium.

In the above-described aspect, the bottom surface of the cell holding container and the top surface of the waste-liquid holding container may adjoin each other, forming a second boundary surface, and the discharge port may open in the second boundary surface.

In the above-described aspect, the cell holding container may have a discharge port, through which the culture medium is discharged, in a side surface thereof, at a predetermined height from the bottom surface, and the discharge mechanism may have a flow path through which the culture medium is discharged from the discharge port to the waste-liquid holding container. Because this configuration allows only the excess culture medium that has exceeded the predetermined height to be discharged, constantly leaving a certain amount of culture medium, it is possible to prevent a decrease in the amount of the culture medium in the cell holding container or exhaustion of the culture medium.

In the above-described aspect, the bottom surface of the cell holding container and the top surface of the waste-liquid holding container may adjoin each other, forming a second boundary surface.

Another aspect of the present invention is a cell culture container having an internal space divided into a culture-medium holding space, a cell holding space, and a waste-liquid holding space in sequence from above in the gravity direction, the cell culture container including: a first partition that divides the culture-medium holding space and the cell holding space; a second partition that divides the cell holding space and the waste-liquid holding space; a first opening that opens in the first partition to communicate between the culture-medium holding space and the cell holding space; and a second opening that opens in the second partition to communicate between the cell holding space and the waste-liquid holding space.

According to this aspect, by gradually replacing the degraded culture medium with an unused culture medium, it is possible to decrease the rate of degradation of the culture medium, enabling culturing of the cells for an extended period of time in a state in which the cell culture container is statically placed in the incubator.

Another aspect of the present invention is a culture-medium changing system including: a cell culture container including, in sequence from above in the gravity direction, a culture-medium holding container that holds a culture medium used to culture cells, a cell holding container that holds the cells and the culture medium supplied from the culture-medium holding container, and a waste-liquid holding container that holds the culture medium discharged from the cell holding container; and a container holder on which the cell culture container is loaded. The cell culture container includes a first tubular member that communicates between the culture-medium holding container and the cell holding container, and a second tubular member that communicates between the cell holding container and the waste-liquid holding container. The culture medium moves from the culture-medium holding container to the cell holding container through the first tubular member, and the culture medium moves from the cell holding container to the waste-liquid holding container through the second tubular member. The container holder includes a base on which the cell culture container is loaded, a first flow-velocity control means that controls the flow velocity of the culture medium flowing inside the first tubular member, a second flow-velocity control means that controls the flow velocity of the culture medium flowing inside the second tubular member, and a controller that controls the first flow-velocity control means and the second flow-velocity control means.

### {Advantageous Effects of Invention}

The present invention can decrease the rate of degradation of a culture medium by gradually replacing a degraded culture medium with an unused culture medium, thus enabling cell culturing for an extended period of time in a state in which a cell culture container is statically placed in an incubator. Thus, it is possible to culture cells having three-dimensional structures without destroying their structures.

### {Brief Description of Drawings}

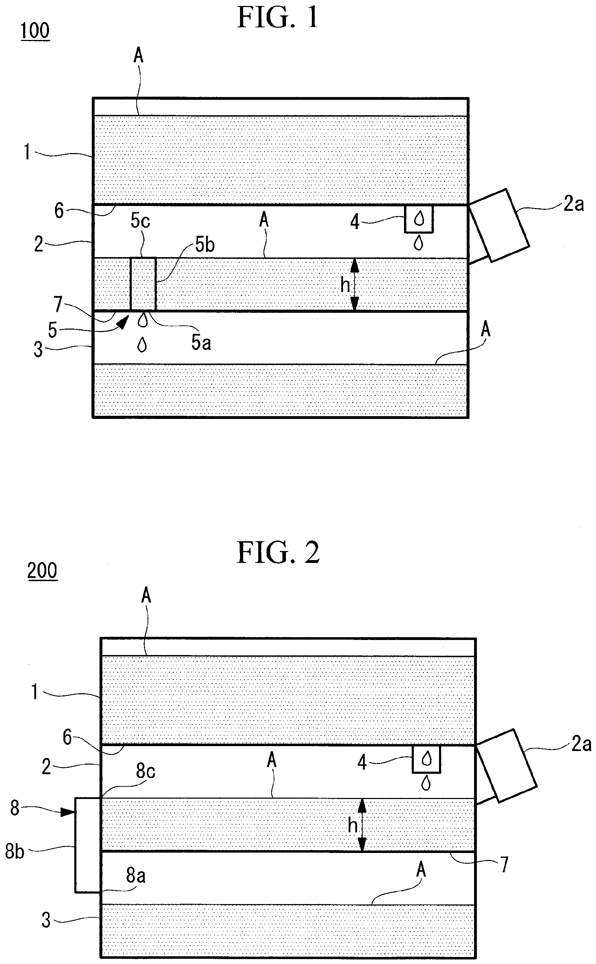

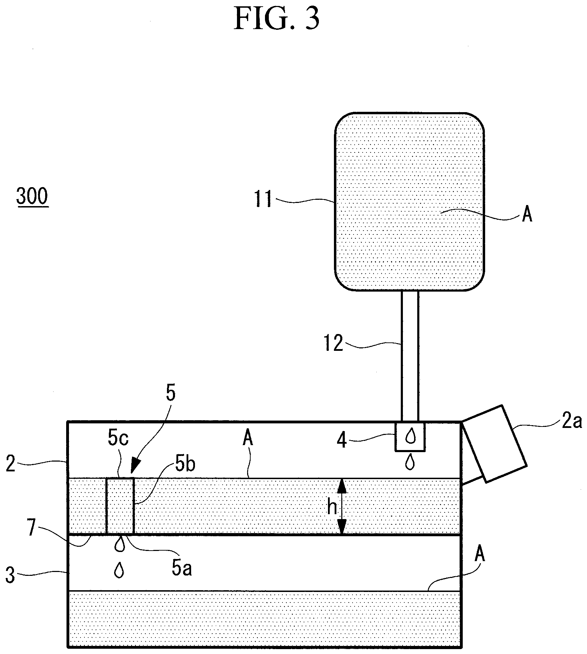

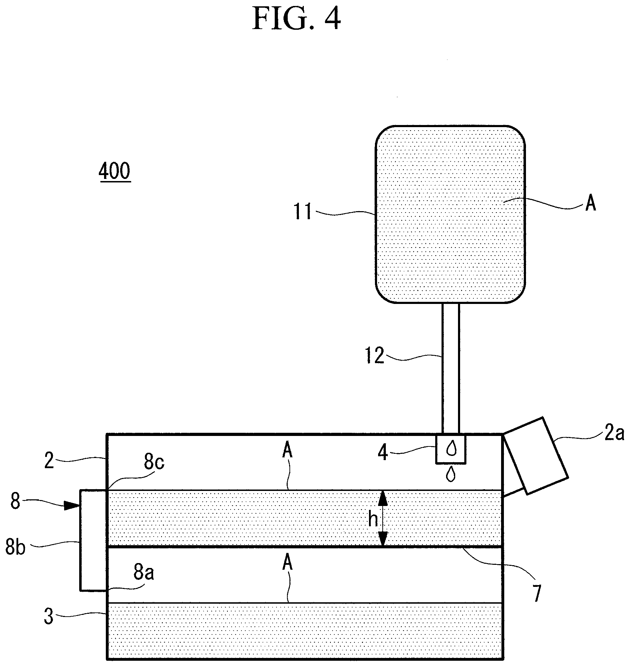

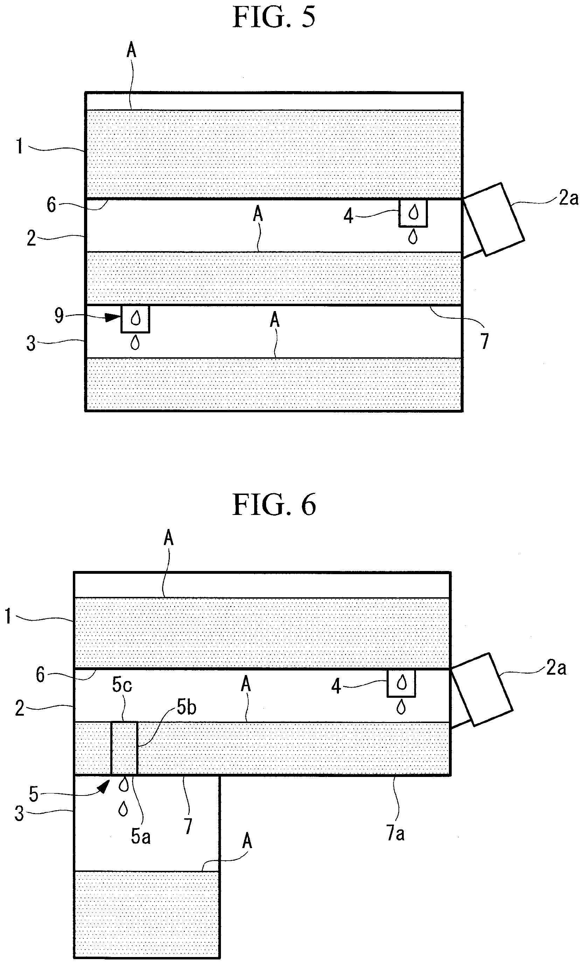

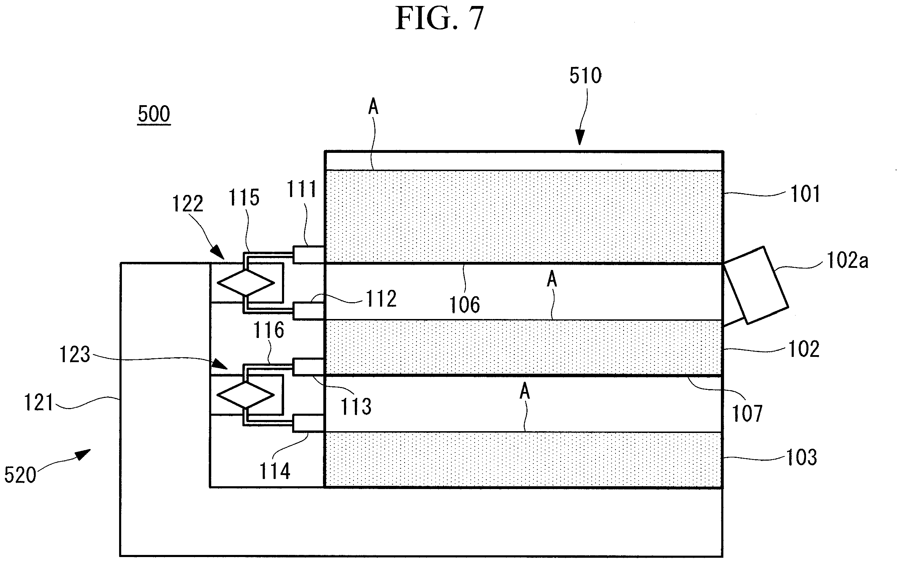

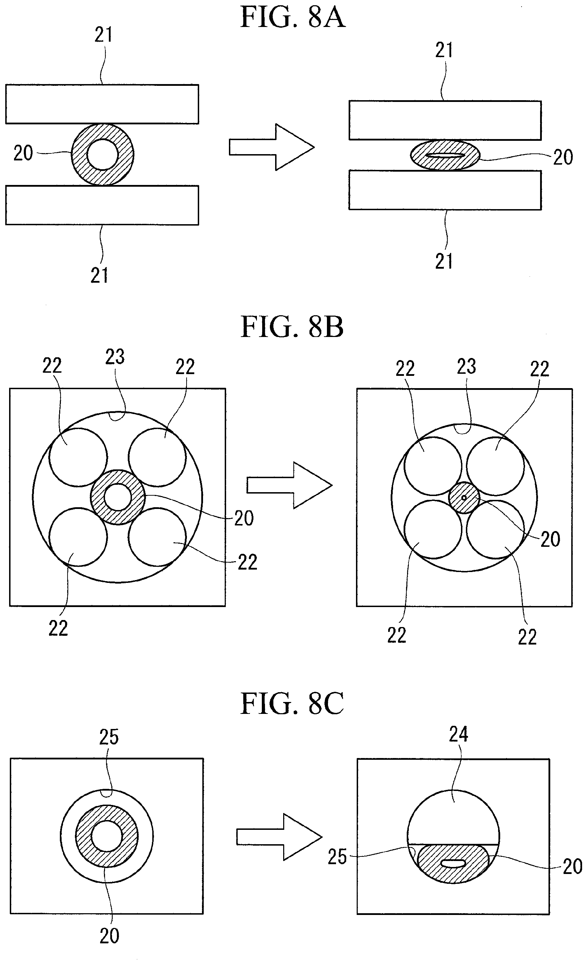

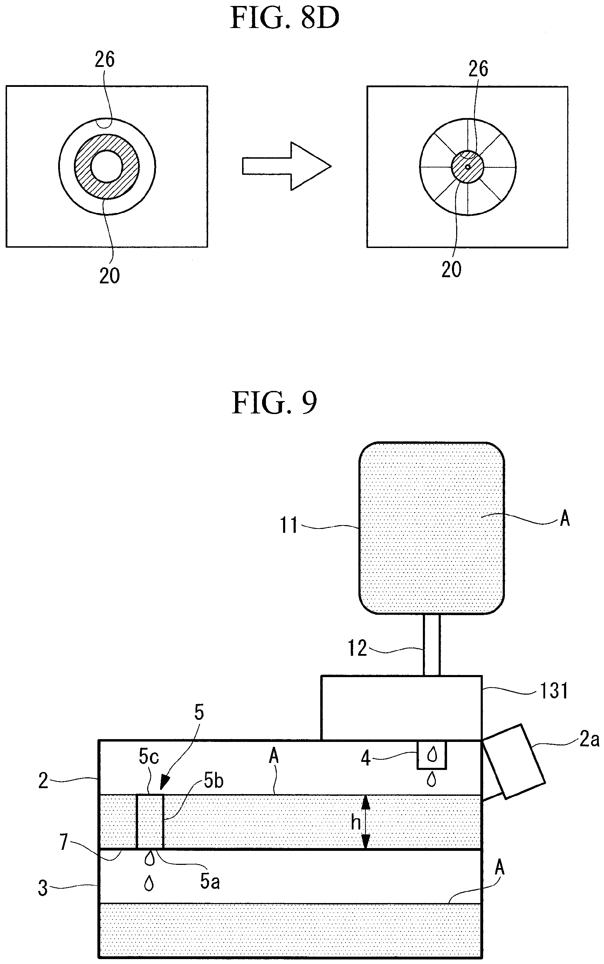

{Fig. 1} Fig. 1 is an explanatory diagram showing, in outline, the configuration of a cell culture container according to a first embodiment of the present invention.
{Fig. 2} Fig. 2 is an explanatory diagram showing, in outline, the configuration of a cell culture container according to a second embodiment of the present invention.
{Fig. 3} Fig. 3 is an explanatory diagram showing, in outline, the configuration of a cell culture container according to a third embodiment of the present invention.
{Fig. 4} Fig. 4 is an explanatory diagram showing, in outline, the configuration of a modification of the cell culture container according to the third embodiment of the present invention.
{Fig. 5} Fig. 5 is an explanatory diagram showing, in outline, the configuration of modifications of the cell culture containers according to the respective embodiments of the present invention.
{Fig. 6} Fig. 6 is an explanatory diagram showing, in outline, the configuration of modifications of the cell culture containers according to the respective embodiments of the present invention.
{Fig. 7} Fig. 7 is an explanatory diagram showing, in outline, the configuration of a culture-medium changing system according to a fourth embodiment of the present invention.
{Fig. 8A} Fig. 8A is an explanatory diagram showing, in outline, the configuration of a flow-velocity control means in the culture-medium changing system in Fig. 7.
{Fig. 8B} Fig. 8B is an explanatory diagram showing, in outline, the configuration of the flow-velocity control means in the culture-medium changing system in Fig. 7.
{Fig. 8C} Fig. 8C is an explanatory diagram showing, in outline, the configuration of the flow-velocity control means in the culture-medium changing system in Fig. 7.
{Fig. 8D} Fig. 8D is an explanatory diagram showing, in outline, the configuration of the flow-velocity control means in the culture-medium changing system in Fig. 7.
{Fig. 9} Fig. 9 is an explanatory diagram showing, in outline, the configuration of a modification of the cell culture container according to the third embodiment of the present invention.

### {Description of Embodiments}

A cell culture container and a culture-medium changing system according to embodiments of the present invention will be described below with reference to the drawings.

### First Embodiment

A cell culture container 100 according to a first embodiment of the present invention is a container having the configuration shown in Fig. 1 and includes a culture-medium holding container 1, a cell holding container 2, and a waste-liquid holding container 3.

The culture-medium holding container 1, the cell holding container 2, and the waste-liquid holding container 3 are arranged so as to adjoin one another in sequence from above in the gravity direction. The culture-medium holding container 1 and the cell holding container 2 are partitioned from each other by a first boundary surface 6, and the cell holding container 2 and the waste-liquid holding container 3 are partitioned from each other by a second boundary surface 7.

The culture-medium holding container 1 can hold an unused culture medium A in the inside thereof and can supply the culture medium A to the cell holding container 2 through a supply port 4 opening in the first boundary surface 6. The culture-medium holding container 1 has an opening (not shown) through which the unused culture medium A is supplied to the inside thereof.

The cell holding container 2 can hold the culture medium A and cells in the inside thereof and has an opening 2a through which the culture medium A and the cells can be inserted and removed.

The culture medium A in the culture-medium holding container 1 is dripped and supplied to the internal space of the cell holding container 2 through the supply port 4. The drip rate is determined by the diameter of the supply port 4. As a result of the culture medium A being dripped into the cell holding container 2, it is possible to prevent backflow of the culture medium A and thus to reduce the possibility of contamination of the culture medium A in the culture-medium holding container 1.

The second boundary surface 7 is provided with a discharge port 5a through which the culture medium A is discharged from the cell holding container 2 to the waste-liquid holding container 3. A tubular member 5b projecting from the discharge port 5a to a predetermined height h toward the upper part of the internal space of the cell holding container 2 and opening at the predetermined height h, forming a flow path, is provided in the cell holding container 2.

When the culture medium A in the cell holding container 2 has reached a predetermined level and exceeded the height h, the culture medium A flows into the tubular member 5b from the opening 5c and is dripped into the waste-liquid holding container 3 through the discharge port 5a. As a result of the culture medium A being dripped into the waste-liquid holding container 3, it is possible to prevent backflow of the culture medium A and thus to reduce the possibility of contamination of the culture medium A in the cell holding container 2. Herein, the discharge port 5a, the tubular member 5b, and the opening 5c serve as a discharge mechanism 5.

The waste-liquid holding container 3 can hold the culture medium A discharged from the cell holding container 2. The waste-liquid holding container 3 may have an opening through which the waste liquid (culture medium A) is discharged from the inside.

Next, an example process of culturing cells using the cell culture container 100 according to this embodiment will be described.

A user of the cell culture container 100 first supplies a culture medium A and cells to be cultured into the cell holding container 2 from the opening 2a. The user also supplies an unused culture medium A into the culture-medium holding container 1 from the opening (not shown) in the culture-medium holding container 1. In this state, the cell culture container 100 is disposed in the incubator, and culture is started.

The unused culture medium A held in the culture-medium holding container 1 is dripped and supplied to the space inside the cell holding container 2 through the supply port 4.

The amount of the culture medium A in the cell holding container 2 increases with time. When the height of the culture medium A in the cell holding container 2 has exceeded h, the excess culture medium flows into the tubular member 5b from the opening 5c and is dripped into the waste-liquid holding container 3 through the discharge port 5a. As a result, the unused culture medium A gradually replaces the degraded culture medium A, decreasing the rate of degradation of the culture medium A.

Note that, by providing the supply port 4 and the discharge port 5a as far away from each other in the horizontal direction as possible, the efficiency of changing the culture medium A can be increased.

### Second Embodiment

A cell culture container 200 according to a second embodiment of the present invention differs from the first embodiment in that, as shown in Fig. 2, it has a discharge mechanism 8 instead of the discharge mechanism 5. The other configurations are the same as those in the first embodiment.

The culture-medium holding container 1, the cell holding container 2, and the waste-liquid holding container 3 are arranged so as to adjoin one another in sequence from above in the gravity direction. The culture-medium holding container 1 and the cell holding container 2 are partitioned from each other by a first boundary surface 6, and the cell holding container 2 and the waste-liquid holding container 3 are partitioned from each other by a second boundary surface 7.

The culture-medium holding container 1 can hold an unused culture medium A in the inside thereof and can supply the culture medium A to the cell holding container 2 through the supply port 4 opening in the first boundary surface 6. The culture-medium holding container 1 has an opening (not shown) through which the unused culture medium A is supplied to the inside thereof.

The cell holding container 2 can hold the culture medium A and cells in the inside thereof and has the opening 2a through which the culture medium A and the cells can be inserted and removed.

The culture medium A in the culture-medium holding container 1 is dripped and supplied to the internal space of the cell holding container 2 through the supply port 4. The drip rate is determined by the diameter of the supply port 4. As a result of the culture medium A being dripped into the cell holding container 2, it is possible to prevent backflow of the culture medium A and thus to reduce the possibility of contamination of the culture medium A in the culture-medium holding container 1.

The cell holding container 2 is provided with, in a side surface thereof, a discharge port 8c through which the culture medium A is discharged, at a predetermined height h from the bottom surface. A tubular member 8b, which forms a flow path extending from the discharge port 8c to the opening 8a in the waste-liquid holding container 3, is provided on the outside of the cell holding container 2.

When the culture medium A in the cell holding container 2 has exceeded the height h, the culture medium A flows into the tubular member 8b from the discharge port 8c and is discharged into the waste-liquid holding container 3 through the opening 8a.

Herein, the opening 8a, the tubular member 8b, and the discharge port 8c serve as a discharge mechanism 8.

The waste-liquid holding container 3 can hold the culture medium A discharged from the cell holding container 2. The waste-liquid holding container 3 may have an opening through which the waste liquid (culture medium A) is discharged from the inside.

Next, an example process of culturing cells using the cell culture container 200 according to this embodiment will be described.

A user of the cell culture container 200 first supplies a culture medium A and cells to be cultured into the cell holding container 2 from the opening 2a. The user also supplies an unused culture medium A from the opening (not shown) in the culture-medium holding container 1. In this state, the cell culture container 200 is disposed in the incubator, and culture is started.

The unused culture medium A held in the culture-medium holding container 1 is dripped and supplied to the space inside the cell holding container 2 through the supply port 4.

The amount of the culture medium A in the cell holding container 2 increases with time. When the height of the culture medium A in the cell holding container 2 has exceeded h, the excess culture medium A flows into the tubular member 8b from the discharge port 8c and is discharged into the waste-liquid holding container 3 through the opening 8a. As a result, the unused culture medium A gradually replaces the degraded culture medium A, decreasing the rate of degradation of the culture medium A.

Note that, by providing the supply port 4 and the discharge port 8c as far away from each other in the horizontal direction as possible, the efficiency of changing the culture medium A can be increased.

### Third Embodiment

A cell culture container 300 according to a third embodiment of the present invention differs from the first embodiment in that, as shown in Fig. 3, it has a culture-medium holding container 11 instead of the culture-medium holding container 1, and the culture medium A is supplied to the cell holding container 2 through a tubular member 12. In all other respects it is the same as the first embodiment.

The culture-medium holding container 11, the cell holding container 2, and the waste-liquid holding container 3 are arranged so as to adjoin one another in sequence from above in the gravity direction. The culture-medium holding container 11 and the cell holding container 2 communicate with each other through the tubular member 12. The cell holding container 2 and the waste-liquid holding container 3 are partitioned from each other by the second boundary surface 7.

The culture-medium holding container 11 can hold an unused culture medium A in the inside thereof and is disposed above the cell holding container 2 in the gravity direction. The culture medium A in the culture-medium holding container 11 is supplied through the tubular member 12 into the cell holding container 2 from the supply port 4 due to gravity.

The cell holding container 2 can hold the culture medium A and cells in the inside thereof and has the opening 2a through which the culture medium A and the cells can be inserted and removed.

The culture medium A in the culture-medium holding container 11 is dripped and supplied to the space inside the cell holding container 2 through the supply port 4. The drip rate is determined by the diameter of the supply port 4. As a result of the culture medium A being dripped into the cell holding container 2, it is possible to prevent backflow of the culture medium A and thus to reduce the possibility of contamination of the culture medium A in the culture-medium holding container 11.

The second boundary surface 7 is provided with the discharge port 5a through which the culture medium A is discharged from the cell holding container 2 to the waste-liquid holding container 3. The tubular member 5b, which forms a flow path projecting from the discharge port 5a to the upper part of the space inside the cell holding container 2, up to a predetermined height h, and opening at the predetermined height h, is provided in the cell holding container 2.

When the culture medium A in the cell holding container 2 has reached a predetermined level and exceeded the height h, the culture medium A flows into the tubular member 5b from the opening 5c and is dripped into the waste-liquid holding container 3 through the discharge port 5a. As a result of the culture medium A being dripped into the waste-liquid holding container 3, it is possible to prevent backflow of the culture medium A and thus to reduce the possibility of contamination of the culture medium A in the cell holding container 2. Herein, the discharge port 5a, the tubular member 5b, and the opening 5c serve as the discharge mechanism 5.

The waste-liquid holding container 3 can hold the culture medium A discharged from the cell holding container 2. The waste-liquid holding container 3 may have an opening through which the waste liquid (culture medium A) is discharged from the inside.

Next, an example process of culturing cells using the cell culture container 300 according to this embodiment will be described.

A user of the cell culture container 300 first supplies a culture medium A and cells to be cultured into the cell holding container 2 from the opening 2a. The user also connects the culture-medium holding container 11, which holds the unused culture medium A therein, to the supply port 4 via the tubular member 12. At this time, the culture-medium holding container 11 is disposed above the cell holding container 2 in the gravity direction. In this state, the cell culture container 300 is disposed in the incubator, and culture is started.

The unused culture medium A held in the culture-medium holding container 11 is dripped and supplied to the internal space of the cell holding container 2 through the supply port 4.

The amount of the culture medium A in the cell holding container 2 increases with time. When the height of the culture medium A in the cell holding container 2 has exceeded h, the excess culture medium A flows into the tubular member 5b from the opening 5c and is dripped into the waste-liquid holding container 3 through the discharge port 5a. As a result, the unused culture medium A gradually replaces the degraded culture medium A, decreasing the rate of degradation of the culture medium A.

Note that, by providing the supply port 4 and the discharge port 5a as far away from each other in the horizontal direction as possible, the efficiency of changing the culture medium A can be increased.

Although the cell culture container 300 according to this embodiment is a modification of the cell culture container 100 according to the first embodiment, it may also be a modification of the cell culture container 200 according to the second embodiment, as shown in Fig. 4. Specifically, it may be a cell culture container 400 that has a culture-medium holding container 11, instead of the culture-medium holding container 1 according to the second embodiment, and that supplies the culture medium A to the cell holding container 2 through the tubular member 12.

In this embodiment, the culture-medium holding container 11 may have an opening (not shown) through which an unused culture medium is supplied to the inside thereof.

Furthermore, the culture-medium holding container 11 and the tubular member 12 may be attachable to and detachable from the supply port 4, and the culture-medium holding container 11 may be attachable to and detachable from the tubular member 12. This configuration enables supply of an unused culture medium by changing the culture-medium holding container 11.

The culture-medium holding container 11 may be a bag.

In the above-described embodiments, the drip rate of the culture medium A supplied to the cell holding container 2 may be appropriately set by the diameter of the supply port 4. The drip rate and the amount of the culture medium A held in the culture-medium holding container 1 may be adjusted depending on the type of the cells to be cultured, the culture conditions, etc.

Alternatively, the drip rate may be set depending on the material of the member constituting the supply port 4.

In the above-described cell culture containers 100 and 200 according to the first embodiment and the second embodiment, the culture-medium holding container 1, the cell holding container 2, and the waste-liquid holding container 3 may be integrally formed. Specifically, the internal spaces of the cell culture containers 100 and 200 may each be divided into, in sequence from above, the culture-medium holding space, the cell holding space, and the waste-liquid holding space. The first opening communicating between the culture-medium holding space and the cell holding space may open in the first partition that divides the culture-medium holding space and the cell holding space, and the second opening communicating between the cell holding space and the waste-liquid holding space may open in the second partition that divides the cell holding space and the waste-liquid holding space.

Alternatively, the culture-medium holding container 1, the cell holding container 2, and the waste-liquid holding container 3 may be formed as independent containers that form the cell culture container 100 or 200 when joined together. In this case, the bottom surface of the culture-medium holding container 1 and the top surface of the cell holding container 2 form the first boundary surface 6, and the bottom surface of the cell holding container 2 and the top surface of the waste-liquid holding container 3 form the second boundary surface 7.

In the cell culture container 300 according to the third embodiment, the cell holding container 2 and the waste-liquid holding container 3 may be integrally formed.

Alternatively, the cell holding container 2 and the waste-liquid holding container 3 may be formed as independent containers that form the cell culture container 300 when joined together. In this case, the bottom surface of the cell holding container 2 and the top surface of the waste-liquid holding container 3 form the second boundary surface 7.

In the above-described embodiments, the cell holding container 2 may have a bottom surface 7a that is not in contact with the waste-liquid holding container 3, as shown in, for example, Fig. 6. In this case, it is desirable that at least the bottom surface 7a be formed of an optically transparent member. This configuration makes it easy to observe the cells in the cell holding container 2 through the bottom surface 7a. Fig. 6 shows a modification of the first embodiment, and this modification can also be equally applied to the second embodiment and the third embodiment.

The cell holding container 2 may have a top surface that is not in contact with the culture-medium holding container 1.

Although the configurations having the discharge mechanisms 5 and 8 for discharging the culture medium A from the cell holding container 2 to the waste-liquid holding container 3 when the culture medium A in the cell holding container 2 has exceeded the predetermined level are shown in the above-described embodiments, a configuration in which an opening 9 is provided in the second boundary surface 7, instead of the discharge mechanisms 5 and 8, to allow the culture medium A to drip from the cell holding container 2 into the waste-liquid holding container 3 through the opening 9, as shown in, for example, Fig. 5 as an example, is also possible. In this case, the drip rate is appropriately set by the diameter of the opening 9. Alternatively, the drip rate may be set depending on the material of the member constituting the opening 9.

### Fourth Embodiment

Next, a culture-medium changing system 500 according to a fourth embodiment of the present invention will be described using Fig. 7.

The culture-medium changing system 500 includes a cell culture container 510 and a container holder 520.

The cell culture container 510 according to this embodiment is a container having the configuration shown in Fig. 7 and includes a culture-medium holding container 101, a cell holding container 102, and a waste-liquid holding container 103.

The culture-medium holding container 101, the cell holding container 102, and the waste-liquid holding container 103 are arranged so as to adjoin one another in sequence from above in the gravity direction. The culture-medium holding container 101 and the cell holding container 102 are partitioned from each other by a first boundary surface 106, and the cell holding container 102 and the waste-liquid holding container 103 are partitioned from each other by a second boundary surface 107.

The culture-medium holding container 101 can hold an unused culture medium A in the inside thereof and has a discharge port 111 that opens in the bottom of a side surface {{I rephrased}} thereof. The discharge port 111 communicates with a supply port 112 that opens in the cell holding container 102 via a tubular member (first tubular member) 115 and can supply the culture medium A from the culture-medium holding container 101 to the cell holding container 102. The culture-medium holding container 101 has an opening (not shown) through which the unused culture medium A is supplied to the inside thereof.

The cell holding container 102 can hold the culture medium A and cells in the inside thereof and has a discharge port 113 that opens in the bottom of a side surface thereof. The discharge port 113 communicates with a supply port 114 that opens in the waste-liquid holding container 103 via the tubular member (second tubular member) 116 and can supply the culture medium A from the cell holding container 102 to the waste-liquid holding container 103. The cell holding container 102 has an opening 102a through which the culture medium A and the cells can be inserted and removed.

The discharge port 113 does not necessarily have to open in the bottom of the side surface, it is only necessary that it open in any position in the side surface of the container 102 at which the culture medium A can be discharged.

The waste-liquid holding container 3 can hold the culture medium A discharged from the cell holding container 2. The waste-liquid holding container 3 may have an opening through which the waste liquid (culture medium A) is discharged from the inside.

According to the cell culture container 510 in this embodiment, the culture medium A in the culture-medium holding container 101 moves to the cell holding container 102 through a tubular member 115 due to gravity, and the culture medium A in the cell holding container 102 moves to the waste-liquid holding container 103 through the tubular member 116 due to gravity.

The container holder 520 includes a base 121 on which the cell culture container 510 is loaded, a flow-velocity control means (first flow-velocity control means) 122 that controls the flow velocity of the culture medium A flowing inside the tubular member 115 of the cell culture container 510, a flow-velocity control means (second flow-velocity control means) 123 that controls the flow velocity of the culture medium A flowing inside the tubular member 116, and a controller (not shown) that is disposed in the base 121 and controls the flow-velocity control means 122 and the flow-velocity control means 123.

The flow-velocity control means 122 and 123 have openings through which the tubular members 115 and 116, respectively, pass. By applying external forces in the radial direction to the tubular members 115 and 116 passing through the openings to deform them, thus reducing the cross-sectional areas of the internal cavities of the tubular members 115 and 116, the flow-velocity control means 122 and 123 limit the flow rates of the solutions to reduce the flow velocities (it is possible to make the flow velocities 0). Conversely, when the external forces are removed, the tubular members 115 and 116 return to the initial states due to the elasticity of the tubular members 115 and 116, increasing the flow velocities. In this way, the flow-velocity control means 122 and 123 adjust the flow velocities of the solutions flowing in the tubular members 115 and 116 by means of the strength of the external forces applied to the tubular members 115 and 116.

An example of how the flow-velocity control means 122 and 123 apply external forces to the tubular members 115 and 116 is shown in Figs. 8A to 8D. Fig. 8A shows an example in which a tubular member 20 (115, 116) is pinched between two platelike members 21. Fig. 8B shows an example in which the tubular member 20 passing through the through-hole 23 is squeezed by a plurality of ball-like (or cylindrical) members 22. Fig. 8C shows an example in which the tubular member 20 passing through the through-hole 25 is pressed by a shutter-like member 24. Fig. 8D is an example in which the inside diameter of the through-hole 26, through which the tubular member 20 passes, is reduced to deform the tubular member 20. Besides these examples, any mechanism that can apply an external force in the radial direction to the tubular member 20 to deform the tubular member 20 may be employed as the flow-velocity control means 122 and 123, and liquid feed pumps may be employed as the flow-velocity control means 122 and 123.

The controller has a timer (not shown) and can control the flow-velocity control means 122 and 123, and thus the culture medium A flowing inside the cell culture container 510, according to a preset time schedule. In other words, it is possible to control the supply of the culture medium to the cell holding container 102 by controlling the flow-velocity control means 122, it is possible to control the discharging of the culture medium from the cell holding container 102 by controlling the flow-velocity control means 123, and thus, it is possible to control changing of the culture medium inside the cell holding container 102. Alternatively, the controller may have a transmitter/receiver (not shown) that transmits/receives a signal to/from an external controller (not shown) via wires or wirelessly and may control the flow-velocity control means 122 and 123 according to signals from the external controller (not shown) to remotely control the culture medium A flowing in the cell culture container 510. An example of the external controller is a personal computer (PC). For example, the PC includes a CPU and a memory and may serve as the external control means by executing a control program stored in the memory with the CPU.

In the above-described fourth embodiment, it is possible to have the discharge mechanism 5 (the discharge port 5a, the tubular member 5b, and the opening 5c) according to the first embodiment, instead of the discharge port 113, the second tubular member 116, the supply port 114, and the second flow-velocity control means 123.

In the above-described third embodiment, as shown in Fig. 9, the tubular member 12 may have a flow-velocity control means 131. The flow-velocity control means 131 has an opening through which the tubular member 12 passes. By applying an external force in the radial direction to the tubular member 12 passing through the opening to deform it, thus reducing the cross-sectional area of the internal cavity of the tubular member 12, the flow-velocity control means 131 limits the flow rate of the solution to reduce the flow velocity (it is possible to make the flow velocity 0). Conversely, when the external force is removed, the tubular member 12 returns to the initial state due to the elasticity of the tubular member 12, increasing the flow velocity. In this way, the flow-velocity control means 131 adjusts the flow velocity of the solution flowing in the tubular member 12 by means of the strength of the external force applied to the tubular member 12. The method of applying an external force to the tubular member 12 with the flow-velocity control means 131 is the same as that with the flow-velocity control means 122 and 123 according to the fourth embodiment. It is also possible to employ, as the flow-velocity control means 131, an arbitrary mechanism that can apply an external force in the radial direction to deform the tubular member 12. For example, a liquid feed pump may be employed as the flow-velocity control means 131.

The flow-velocity control means 131 has a timer (not shown) and a controller (not shown) and can control the culture medium A flowing in the tubular member 12 according to a preset time schedule. Alternatively, the controller (not shown) may have a transmitter/receiver (not shown) that transmits/receives a signal to/from an external controller (not shown) via wires or wirelessly and may control the culture medium A flowing in the tubular member 12 according to the signal from the external controller (not shown). An example of the external controller is a personal computer (PC). For example, the PC includes a CPU and a memory and may serve as the external control means by executing a control program stored in the memory with the CPU.

The present invention can provide a culture-medium changing system including a cell culture container and a container holder on which the cell culture container is loaded. The cell culture container includes, in sequence from above in the gravity direction: a culture-medium holding container that holds a culture medium used to culture cells; a cell holding container that holds the cells and the culture medium supplied from the culture-medium holding container; and a waste-liquid holding container that holds the culture medium discharged from the cell holding container. The cell culture container also includes a first tubular member that communicates between the culture-medium holding container and the cell holding container, and a second tubular member that communicates between the cell holding container and the waste-liquid holding container. The culture medium moves from the culture-medium holding container to the cell holding container through the first tubular member due to gravity, and the culture medium moves from the cell holding container to the waste-liquid holding container through the second tubular member due to gravity. The container holder includes a base on which the cell culture container is loaded, a first flow-velocity control means that controls the flow velocity of the culture medium flowing inside the first tubular member, a second flow-velocity control means that controls the flow velocity of the culture medium flowing inside the second tubular member, and a controller that controls the first flow-velocity control means and the second flow-velocity control means.

The present invention can provide a cell culture container that includes: a culture-medium holding container that holds a culture medium used to culture cells; a cell holding container that holds the cells and the culture medium supplied from the culture-medium holding container; and a waste-liquid holding container that holds the culture medium discharged from the cell holding container.

### {Reference Signs List}

1, 11 culture-medium holding container
2 cell holding container
3 waste-liquid holding container
4 supply port
5, 8 discharge mechanism
5a discharge port
5b tubular member (flow path)
6, 7 boundary surface
12 tubular member
100, 200, 300, 400 cell culture container
500 culture-medium changing system

## Claims

1. A cell culture container comprising:
a culture-medium holding container that holds a culture medium used to culture cells;
a cell holding container that has, in the top surface thereof, a supply port through which the culture medium is supplied from the culture-medium holding container and that holds the cells and the culture medium supplied from the culture-medium holding container through the supply port;
a waste-liquid holding container that holds the culture medium discharged from the cell holding container; and
a discharge mechanism for discharging the culture medium from the cell holding container to the waste-liquid holding container when the culture medium in the cell holding container has exceeded a predetermined level.

2. The cell culture container according to Claim 1, wherein
the bottom surface of the culture-medium holding container and the top surface of the cell holding container adjoin each other, forming a boundary surface, and
the supply port opens in the boundary surface.

3. The cell culture container according to Claim 1, wherein
the culture-medium holding container communicates with the supply port via a tubular member, and
the culture-medium holding container is disposed above the cell holding container in the gravity direction.

4. The cell culture container according to Claim 3, wherein the culture-medium holding container is a bag attachable to and detachable from the supply port.

5. A cell culture container according to any one of Claims 1 to 4, wherein
the cell holding container has, in the bottom surface thereof, a discharge port through which the culture medium is discharged from the cell holding container to the waste-liquid holding container, and
the discharge mechanism includes a flow path projecting from the discharge port to the upper part of the space inside the cell holding container and opening at a predetermined height.

6. The cell culture container according to Claim 5, wherein the bottom surface of the cell holding container and the top surface of the waste-liquid holding container adjoin each other, forming a second boundary surface, and the discharge port opens in the second boundary surface.

7. The cell culture container according to any one of Claims 1 to 4, wherein
the cell holding container has a discharge port, through which the culture medium is discharged, in a side surface thereof, at a predetermined height from the bottom surface, and
the discharge mechanism has a flow path through which the culture medium is discharged from the discharge port to the waste-liquid holding container.

8. The cell culture container according to Claim 7, wherein the bottom surface of the cell holding container and the top surface of the waste-liquid holding container adjoin each other, forming a second boundary surface.

9. A cell culture container having an internal space divided into a culture-medium holding space, a cell holding space, and a waste-liquid holding space in sequence from above in the gravity direction, comprising:
a first partition that divides the culture-medium holding space and the cell holding space;
a second partition that divides the cell holding space and the waste-liquid holding space;
a first opening that opens in the first partition to communicate between the culture-medium holding space and the cell holding space; and
a second opening that opens in the second partition to communicate between the cell holding space and the waste-liquid holding space.

10. A culture-medium changing system comprising:
a cell culture container including, in sequence from above in the gravity direction, a culture-medium holding container that holds a culture medium used to culture cells, a cell holding container that holds the cells and the culture medium supplied from the culture-medium holding container, and a waste-liquid holding container that holds the culture medium discharged from the cell holding container; and
a container holder on which the cell culture container is loaded, wherein
the cell culture container includes a first tubular member that communicates between the culture-medium holding container and the cell holding container, and a second tubular member that communicates between the cell holding container and the waste-liquid holding container,
the culture medium moves from the culture-medium holding container to the cell holding container through the first tubular member, and the culture medium moves from the cell holding container to the waste-liquid holding container through the second tubular member, and
the container holder includes a base on which the cell culture container is loaded, a first flow-velocity control means that controls the flow velocity of the culture medium flowing inside the first tubular member, a second flow-velocity control means that controls the flow velocity of the culture medium flowing inside the second tubular member, and a controller that controls the first flow-velocity control means and the second flow-velocity control means.
